# DEMANDE DE BREVET EUROPEEN

(11) **EP 1 475 081 A1**
(43) Date de publication de la demande: **10.11.2004**
(21) Numéro de dépôt: 04291134.7
(22) Date de dépôt: 04.05.2004
(51) Int. Cl.: A61K 7/48, A61K 7/027, A61K 7/031, A61K 7/032, A61K 7/043, A61K 7/42

(54) **Composition cosmétique pour le soin et/ou le maquillage de la peau, des lèvres et des phanères.**

(30) Priorité: 05.05.2003 FR 0305447
(71) Demandeur: L'OREAL, 75008 Paris (FR)
(72) Inventeur: Mondet, Jean, 93600 Aulnay sous Bois (FR); Ilekti, Philippe, 75003 paris (FR)
(74) Mandataire: Le Coupanec, Pascale

(57) **Abrégé**

La présente invention concerne une composition cosmétique de soin et/ou de maquillage de la peau, des lèvres et/ou des phanères comprenant, dans un milieu physiologiquement acceptable contenant au moins une phase grasse, au moins une quantité efficace de phyllosilicates exfoliés dérivant d'au moins un phyllosilicate intercalé par une ou plusieurs molécules d'un agent intercalant non polyphénolique.

## Description

La présente invention concerne une composition cosmétique pour le soin et/ou le maquillage de la peau, des lèvres et des phanères.

Plus particulièrement, les compositions considérées selon l'invention, peuvent constituer un produit de maquillage de la peau, des lèvres et/ou des phanères ayant notamment des propriétés de soin et/ou de traitement non thérapeutique.

La présente invention vise notamment à améliorer la persistance d'un effet cosmétique d'une composition cosmétique, notamment pour le maquillage de la peau, y compris des paupières, ainsi que des lèvres, des phanères comme les cils et les sourcils, en particulier les ongles. Les produits cosmétiques plus particulièrement concernés sont par exemple, les rouges à lèvres, mascaras, eye-liners, fonds de teint, poudres, fards à joues, ombres à paupières, vernis à ongles, produits de soin des ongles, et les produits de maquillage du corps.

Classiquement, les compositions de maquillage précitées sont appliquées sur la surface à maquiller sous la forme d'un dépôt destiné à procurer les effets de coloration et/ou de brillance et/ou de matité attendus.

Par définition, un produit matifiant est un produit qui unifie le teint. Les compositions du soin de la peau ou de maquillage dotées de propriétés matifiantes sont généralement utilisées pour résoudre les problèmes de brillance occasionnés par un excès de sébum et pour améliorer la tenue du maquillage à long terme qui a tendance à se dégrader visuellement au cours de la journée. Ces compositions matifiantes confèrent généralement un aspect mat à la peau résultant d'un pouvoir de diffusion de la lumière à la surface de la peau. Elles sont également avantageuses pour estomper les défauts cutanés tels que microreliefs, rides, ridules, pores ou variations de couleurs.

Les effets de coloration et de brillance sont des effets esthétiques plus particulièrement recherchés pour les rouges à lèvres, les mascaras et les eye-liners. Quant à l'effet de matité, il concerne plus particulièrement les compositions du soin ou de maquillage de la peau.

Pour des raisons évidentes, il est souhaitable que les effets cités précédemment perdurent le plus longtemps possible dans le temps. Par exemple, pour les rouges à lèvres, les mascaras et les eye-liners, il est important d'obtenir une durée prolongée de la coloration et/ou de la brillance. Pour les fonds de teint, poudres, fards à joues, ombres à paupières et les produits de maquillages du corps, il est important d'obtenir un effet coloré et mat, persistant et durable, et ceci en dépit des agressions de type frottements et/ou sécrétions de sébum ou de sueur subie par la couche de maquillage appliquée.

Pour ce faire, les produits cosmétiques et notamment de maquillage contiennent, outre une phase grasse telle que cire et/ou huile, au moins une charge généralement minérale. Il est ainsi connu d'introduire des charges dans les compositions cosmétiques afin d'ajuster leurs propriétés en terme de texture et notamment pour améliorer leurs propriétés en terme de tenue. Les charges minérales ou organiques classiquement utilisées sont par exemple la silice, des poudres de nylon ou encore la bentone qui est un matériau phyllosilicate dont les surfaces externes de ses empilements de feuillets ont été traitées de manière à les rendre organophiles. Pour leur part, les compositions matifiantes contiennent généralement des poudres absorbant le sébum et l'huile excédentaire de la composition non absorbée par la peau. Parmi les poudres matifiantes d'origine naturelle ou synthétique, on peut citer notamment certaines des charges précédentes comme le talc, la silice et les poudres de nylon, mais également l'amidon, le mica, les poudres de polyéthylène et les poudres de poly(méth)acrylate de méthyle.

En fait, la taille de ces charges conventionnelles, généralement à l'échelle du micron, et/ou leur matité naturelle peuvent affecter les propriétés esthétiques des compositions cosmétiques les incorporant en quantité significativement importante.

Ainsi, dans le cas de certaines compositions cosmétiques comme les rouges à lèvres par exemple, il est nécessaire d'utiliser une quantité importante de charges pour pouvoir limiter suffisamment la migration des huiles dans les ridules de la peau et en particulier des lèvres et/ou pour obtenir une bonne tenue. La matité de ces charges peut donc provoquer dans ces conditions une perte significative de brillance de la composition cosmétique correspondante.

De même dans le cas des compositions matifiantes, on note très souvent un aspect poudreux, conséquence d'une taille trop importante desdites charges, qui est incompatible avec un effet naturel.

D'une manière inattendue, les inventeurs ont constaté qu'il était possible de surmonter cet inconvénient tout en garantissant une tenue convenable et prolongée dans le temps aux compositions cosmétiques, sous réserve du choix d'une charge spécifique.

En l'occurrence, les inventeurs ont découvert que les phyllosilicates intercalés et exfoliés constituaient un additif particulièrement intéressant dans le domaine de la cosmétique pour accéder à des compositions cosmétiques cumulant avantageusement de bonnes propriétés de texture, de tenue, de brillance, d'absence de migration et de matité.

Les phyllosilicates, plus communément appelés argiles, désignent d'une manière générale des silicates en feuillets.

Le terme « phyllosilicates intercalés », signifie que des phyllosilicates ont été traités avec des composés organiques ou inorganiques en vue d'introduire des molécules de ces composés dans les espaces interfoliaires desdits phyllosilicates d'une part pour augmenter la distance entre les feuillets et d'autre part, pour leur apporter un caractère organophile. Généralement, une quantité suffisante de molécules est adsorbée entre deux feuillets adjacents de phyllosilicates de manière à augmenter leur espace interfoliaire à une dimension d'au moins 5 angströms, en particulier d'environ 10 angströms et à favoriser ainsi l'exfoliation consécutive du matériau intercalé sous la forme de feuillets séparées. Cette exfoliation, ou encore délamination, est généralement réalisée sous cisaillement en présence d'un solvant organique ou dans une matrice polymérique de manière à obtenir des « phyllosilicates exfoliés ».

A ce jour, deux techniques d'intercalation sont plus particulièrement utilisées.

La première technique notamment décrite dans le document WO 93/04118 consiste à échanger les cations minéraux hydrophiles, originellement présents entre les feuillets de phyllosilicates, par des cations « onium » organiques, généralement des alkylammoniums quaternaires. Les phyllosilicates ainsi intercalés sont utilisés sous leur forme exfoliée dans des matrices polymériques en vue de renforcer notamment leurs propriétés mécaniques. Les cations d'ammonium quaternaire sont en particulier bien connus pour convertir des phyllosilicates très hydrophiles tels que les montmorillonites de calcium ou de sodium en phyllosilicates organophiles.

La seconde technique, vise à modifier la nature des ligands auxquels sont coordonnés les cations des espaces interfoliaires. A l'état naturel, les cations des espaces interfoliaires sont coordonnés à des molécules d'eau. Cette seconde méthode vise à substituer à ces molécules d'eau des molécules hydrocarbonées organiques spécifiques comportant au moins un groupe polaire. Ainsi, Le brevet US 5 721 306 propose plus particulièrement à titre d'agent intercalant des molécules hydrocarbonées possédant au moins un groupement polaire de type hydroxyle, carbonyle, carboxyle, amine, amide, éther, ester, et voire aussi sulfate, sulfonate, sulfinate, sulfamate, phosphate, phosphonate ou phosphinate, ou un groupe aromatique, capable d'interagir avec les cations métalliques liés aux surfaces des feuillets de phyllosilicates. Il s'agit notamment de dérivés d'alkylpyrrolidone, de polyvinylpyrrolidone, de polyvinylalcool, de polyoxyalkylènes, de polyamide ou de polyimide. Les phyllosilicates ainsi intercalés, sont exfoliés ultérieurement. Ils sont notamment proposés dans ce document, comme agent épaississant pour préparer des formulations de type gel thixotropique visqueux, et en particulier à titre de véhicules pour une grande variété de matières actives. Plus récemment, le brevet US 6 500 411, propose d'utiliser des phyllosilicates intercalés et exfoliés à l'aide de dérivés polyphénoliques naturels, à l'image de la lignine par exemple, dans des compositions cosmétiques aqueuses notamment de type formulation solaires.

La présente invention concerne, selon l'un de ses premiers aspects une composition cosmétique de soin et/ou de maquillage de la peau, des lèvres et/ou des phanères comprenant un milieu physiologiquement acceptable contenant au moins une phase grasse et au moins une quantité efficace de phyllosilicates exfoliés dérivant d'au moins un phyllosilicate intercalé par une ou plusieurs molécules d'un agent intercalant non phénolique.

Dans l'expression «phyllosilicates exfoliés dérivant d'au moins un phyllosilicate intercalé », on entend par le terme « dérivant », qualifié le fait que les phyllosilicates à l'état de feuillets sont obtenus à partir de phyllosilicates intercalés par application à ces derniers d'une force de cisaillement suffisante pour permettre au moins en partie, et notamment en totalité leur exfoliation. Le type de force de cisaillement susceptible d'être appliquée est plus particulièrement détaillé ci-après.

Selon un mode de réalisation particulier de l'invention, le milieu physiologiquement acceptable contient au moins une phase grasse liquide.

En particulier, la composition est une émulsion.

Plus particulièrement, un autre aspect de l'invention concerne une composition cosmétique de soin et/ou de maquillage de la peau, des lèvres et/ou des phanères de type émulsion, ladite composition comprenant, dans un milieu physiologiquement acceptable contenant au moins une phase grasse, au moins une quantité efficace de phyllosilicates exfoliés dérivant d'au moins un phyllosilicate intercalé par une ou plusieurs molécules d'un agent intercalant et étant dépourvue d'agent émulsionnant et d'agent co-émulsionnant.

De manière avantageuse, les émulsions contenant les phyllosilicates exfoliés conformes à l'invention peuvent contenir une moindre quantité d'agents émulsionnants et d'agents co-émulsionnants, voire être totalement exemptes de ces agents.

Les inventeurs ont ainsi constaté que les phyllosilicates conformes à l'invention pouvaient être avantageusement mis à profit à titre d'agent stabilisant pour assurer la stabilité d'émulsions cosmétiques, qui d'ordinaire requiert une quantité efficace en tensioactifs(s) ou en agents émulsionnants et le cas échéant en agents co-émulsionnants. Les phyllosilicates permettent de réduire significativement, voire de s'affranchir totalement de la présence de ces tensioactifs classiques, ou agents émulsionnants et co-émulsionnants, usuellement requis avec un avantage évident sur le plan de l'innocuité.

Avantageusement, les compositions selon l'invention peuvent être transparentes ou translucides et/ou susceptibles de donner un dépôt transparent ou translucide.

Au sens de l'invention, cette propriété de transparence ou de translucidité « dans la masse » signifie qu'une couche de la composition d'une épaisseur donnée, laisse passer une partie de la lumière visible. Si cette partie de la lumière visible est diffusée, la composition sera définie comme étant une composition translucide dans la masse, et si, au contraire, elle n'est pas diffusée, alors la composition sera définie comme étant une composition transparente dans la masse.

La présente invention vise plus précisément la valorisation de phyllosilicates intercalés puis exfoliés dans des compositions cosmétiques spécifiques.

La présente invention a également pour objet, selon un autre de ses aspects, l'utilisation de phyllosilicates exfoliés conformes à l'invention à titre d'agent pour ajuster les propriétés de tenue et notamment la non-migration et le non-transfert d'une composition cosmétique de maquillage et/ou de soin pour la peau, les lèvres et/ou les phanères.

La présente invention a encore pour objet, selon un autre de ses aspects, l'utilisation de phyllosilicates exfoliées conformes à l'invention pour stabiliser des émulsions notamment phase aqueuse-dans-phase huileuse ou phase huileuse-dans-phase aqueuse.

La présente invention a également pour objet, selon un autre de ses aspects, l'utilisation de phyllosilicates exfoliés conformes à l'invention à titre d'agent pour texturer la phase grasse liquide d'une composition cosmétique de soin et/ou de maquillage de la peau, des lèvres et/ou des phanères.

Dans le cadre de la présente invention, les phyllosilicates exfoliés sont dénués de toute activité de véhicule à l'égard de tout actif susceptible d'être présent dans ladite composition cosmétique. En d'autres termes, ils n'ont pas vocation à participer au conditionnement d'une substance active dans la composition cosmétique.

La présente invention a encore pour objet, un procédé de maquillage et/ou de soin de la peau, des lèvres, des ongles, et/ou des phanères comprenant l'application sur la peau, les lèvres, les ongles, et/ou les phanères d'au moins une composition conforme à la présente invention.

Selon un autre de ses aspects, l'invention a encore pour objet un support synthétique, notamment de type faux cils, sur lequel est présent en tout ou partie de sa surface au moins une couche d'une composition selon l'invention.

### PHYLLOSILICATES EXFOLIES

Comme précisé précédemment, les phyllosilicates exfoliés dérivent d'au moins un phyllosilicate intercalé peuvent être présents dans la composition cosmétique selon l'invention sous divers degrés d'exfoliation.

Ainsi certains phyllosilicates exfoliés peuvent être présents sous une forme totalement exfoliée c'est-à-dire sous la forme d'un unique feuillet et d'autres en revanche sous une forme partiellement exfoliée c'est-à-dire sous une forme comprenant, deux ou plus, feuillets encore associés. Avantageusement, au moins 50 %, notamment 70 %, en particulier 90 %, voire 95 % de ces phyllosilicates exfoliés peuvent être formés de moins de 10 feuillets, notamment moins de 5 feuillets, en particulier moins de 3 feuillets, voire d'un seul feuillet de phyllosilicate.

L'ensemble de ces formes peut bien entendu coexister dans la composition cosmétique selon l'invention voire en mélange avec des phyllosilicates intercalés non exfoliés.

Les phyllosilicates exfoliés selon l'invention peuvent être caractérisés par un facteur de forme qui est le rapport de la plus grande dimension sur la plus petite dimension. Ce facteur de forme peut varier de 50 à 2000, notamment de 75 à 1500, et en particulier de 200 à 1000.

Les phyllosilicates exfoliés selon l'invention peuvent présenter une épaisseur moyenne supérieure à 5 Å et une épaisseur maximum inférieure à 100 Å, et en particulier variant de 10 Å à 50 Å.

Compte tenu de leur faible épaisseur, à l'échelle de quelques angströms, les phyllosilicates exfoliés, avantageusement, n'apportent pas d'opacité contrairement aux charges conventionnelles. En effet, à l'image des compositions matifiantes classiques, les compositions selon l'invention et notamment celles destinées à être appliquées sur la peau, comme le fond de teint, s'avèrent avantageusement efficaces pour unifier le teint et notamment estomper les imperfections cutanées tout en procurant un aspect naturel à la peau maquillée. De telles compositions possèdent un bon pouvoir diffusant de la lumière en surface, stable dans le temps.

Ces phyllosilicates permettent par ailleurs de conférer à une composition cosmétique des propriétés intéressantes en terme de tenue sans pour autant porter préjudice à ses qualités esthétiques telles effet coloré, brillance et matité.

De même, les phyllosilicates peuvent être utilisés pour assurer la stabilisation d'émulsion et, à ce titre, être utilisés efficacement en substitution et/ou remplacement total ou partiel des tensioactifs généralement requis pour assurer ce type de stabilisation.

Les phyllosilicates utilisables selon l'invention, dérivent plus particulièrement de l'intercalation d'argiles minérales de type smectite comme les montmorillonites notamment les montmorillonites de sodium, de potassium et/ou de calcium, les nontronites, beidellites, volkonskoïtes, hectorites, saponites, sauconites, sobockites, stevensites, svinfordites, vermiculites et leurs mélanges. Conviennent tout particulièrement à l'invention les phyllosilicates de type montmorillonite.

Les phyllosilicates exfoliés selon l'invention présentent, avantageusement adsorbés à la surface de leurs feuillets, au moins un agent intercalant.

Lequel agent était intercalé entre les feuillets du phyllosilicate dit « intercalé » utilisé pour préparer la composition selon l'invention.

Les phyllosilicates exfoliés possèdent en particulier au moins 15 % en poids, notamment au moins 20 % en poids et plus particulièrement au moins 30 % en poids d'agent intercalant par rapport au poids de phyllosilicate sec (comprenant moins de 5 % d'eau).

Comme précisé précédemment, les phyllosilicates exfoliés selon l'invention sont exempts d'agent intercalant polyphénolique.

Au sens de la présente invention, sont notamment exclus sous ce terme les agents intercalants naturels, comprenant au moins un motif phénolique à l'image par exemple de la lignine, les lignosulfonates, les humates, les tanates, leurs sels et dérivés.

Les agents intercalants peuvent être adsorbés sur tout ou partie de la surface des feuillets de phyllosilicate, et notamment présenter avec ledit phyllosilicate ou des cations présents à sa surface des liaisons de type hydrogène, ionique, covalente et/ou des interactions hydrophiles, de Van der Waals.

D'une manière générale, les agents intercalants considérés selon l'invention possèdent dans leur structure chimique au moins un groupement hydrophobe, notamment une chaîne alkyle en C₄ à C₅₀, alkylène en C₄ à C₅₀ et/ou alkylaryle en C₄ à C₅₀.

Dans le cadre de la présente invention, les agents intercalants plus particulièrement considérés sont des composés choisis parmi :
- les oniums possédant au moins une chaîne hydrocarbonée en C₁-C₅₀, notamment en C₄-C₅₀, et
- les agents intercalants dits à groupe polaire comme les composés organiques, de nature polymérique ou non, possédant au moins un cycle aromatique ou au moins un groupe polaire choisi parmi les groupes carbonyle, hydroxyle, polyol (dont glycol, glycerol, etc.), acide carboxylique, aldéhyde, cétone, amine, amide (linéaire ou cyclique en particulier pyrrolidone, caprolactame), ester, lactone, éther, voire sulfate, sulfonate, sulfinate, sulfamate, phosphate, phosphonate et phosphinate.

Comme indiqué précédemment, les agents intercalants du type onium remplacent en partie ou totalement les ions minéraux hydrophiles (Na+, K+, ...) présents dans les phyllosilicates à l'état naturel. On parle encore d'intercalation par échange d'ions. Quant aux agents intercalants possédant au moins un groupe polaire, ils remplacent en partie ou totalement les molécules d'eau qui coordonnaient initialement les cations métalliques hydrophiles présents entre les feuillets.

### Agent intercalant de type onium

Par « oniums », on désigne les groupes ammonium, sulfonium ou phosphonium tel qu'il est indiqué par exemple dans la demande WO 93/04118 de ALLIED SIGNAL.

Parmi ces oniums, on peut citer plus particulièrement les ammoniums primaires N⁺H₃R₁, les ammoniums secondaires, les ammoniums tertiaires et les ammoniums quaternaires et notamment de type N⁺H₂R₁R₂, N⁺HR₁R₂R₃ et N⁺R₁R₂R₃R₄ dans lesquels les groupes R₁, R₂, R₃, R₄, égaux ou différents représentent des chaînes hydrocarbonées en C₁-C₅₀, dont une au moins est plus particulièrement en C₄-C₅₀, ces chaînes hydrocarbonées pouvant être linéaires, ramifiées ou cycliques, saturées ou insaturées et pouvant comporter un ou plusieurs hétéroatomes tels que O, S, N, Si ou P. Il peut notamment s'agit de chaînes alkyle en C₄ à C₅₀, alkylène en C₄ à C₅₀ ou alkylaryle en C₄ à C₅₀.

Selon un aspect de la présente invention, l'un des substituants R₁ à R₄ du groupe omnium peut être de nature aromatique (benzyle ou phényle) ou alkyl aryle, les autres groupes étant alors des chaînes alkyles en C₄ à C₅₀ comme défini précédemment.

A titre illlustratif et non limitatif des agents intercalants de type onium utilisables selon l'invention, on peut notamment citer ceux décrits dans la demande WO 93/04118 de ALLIED SIGNAL, les agents intercalants di- ou multi- oniums décrits dans EP 1 038 834 et WO 00/09605 de AMCOL et les agents intercalants à groupe onium, de préférence ammoniums primaires, secondaires ou tertiaires possédant deux chaînes alkyles en C ≥ 10 tels que décrits dans JP 04 357108 de NIPPON PAINT.

Parmi ces oniums, conviennent tout particulièrement les ammoniums primaires, secondaires, tertiaires ou quaternaires, de préférence quaternaires et possédant au moins une chaîne alkyle en C₄ à C₁₀, chaîne qui est de préférence linéaire ou ramifiée et de préférence saturée, comme par exemple une chaîne butyle, isobutyle, pentyle, isopentyle, hexyle, heptyle, éthyl 2-hexyle, octyle, nonyle, décyle, undécyle, dodécyle, ....octadécyle. Parmi ces cations ammoniums, conviennent tout particulièrement ceux qui possèdent au moins une chaîne alkyle de type dodécyle ou octadécyle.

### Agents intercalants à groupe polaire

Parmi les agents intercalants à groupe polaire, on distinguera :
a) ceux qui ne sont pas de nature polymérique,
b) ceux qui sont de nature polymérique.

Au sens de la présente invention, on désigne sous le terme de « composé de nature polymérique », un composé possédant au moins deux motifs de répétition, notamment au moins trois motifs de répétition, en particulier au moins dix motifs de répétition voire au moins quinze motifs de répétition. Il peut être par ailleurs composé d'un unique motif répétitif (homopolymère) ou d'au moins deux motifs répétitifs de nature différente (copolymère).

### a) Agents intercalants à groupe polaire de nature non polymérique

Ils comportent au moins un groupe polaire tel que défini ci-dessus et au moins, de préférence une chaîne hydrophobe notamment hydrocarbonée en C₄-C₅₀, qui peut être linéaire, ramifiée ou cyclique, saturée ou non et peut en plus contenir des hétéroatomes tels que O, S, N, Si ou P. Il peut s'agir notamment d'une chaîne alkyle en C₄-C₅₀, alkylène en C₄-C₅₀ ou alkyle aryle en C₄-C₅₀. De tels composés sont notamment décrits dans les documents US 5 721 306, EP 780 340 et US 6 242 500.

A titre illustratif de ce type de composé, on peut notamment citer ceux comprenant à titre de groupe polaire, au moins un groupe choisi parmi les groupes alcools C₆₋₂₄, les glycérols ayant au moins une chaîne en C₆-C₂₄, les acides carboxyliques en C₆-C₂₄ notamment ceux décrits dans EP 780 340, les groupes amides, de préférence cycliques à l'image des lactames tels que les dérivés pyrrolidones ou caprolactames et substitués par un groupe aromatique, comme ceux décrits dans US 6 242 500, ou par une chaîne alkyle en C₄-C₅₀, de préférence en C₈-C₃₀, de préférence en C₁₂-C₂₅ comme par exemple les alkylpyrrolidones avec une chaîne alkyle en C₄ à C₅₀, en particulier en C₈ à C₃₀. Il peut notamment s'agir de la dodécyl pyrrolidone décrite en particulier dans *« Nanocomposites* *produces utilizing a novel dipole clay surface modification in polymer-clay composites* », BEALL G.W., Chemistry and Technology of Polymer Additives (1999), 266-280, Editor : Al-Malaika, Sahar ; Publisher : Blackwell, Oxford UK.

### b) Agents intercalants à groupe polaire de nature polymérique

Il s'agit plus particulièrement d'oligomères ou d'homo- ou co-polymères synthétiques qui comportent au moins un noyau aromatique ou un groupe polaire tel que défini ci-dessus. Le PM peut varier de 300 à 200 000, et en particulier de 500 à 40 000. De tels agents sont notamment décrits dans US 5 837 763.

Ces oligomères ou polymères peuvent être hydrophiles ou hydrophobes.

A titre représentatif de ces agents intercalants polymériques hydrophiles, on peut notamment citer les dérivés polyvinylpyrrolidones (PVP), les dérivés polyvinylalcools (PVA), notamment lorsque ceux-ci sont quasiment sous leur forme acétate de polyvinyle hydrolysée ou en d'autres termes possèdent moins de 5 % de groupes acétyles résiduels, les dérivés polyacryliques sous leur forme polymérique et copolymérique et plus particulièrement sous forme de leurs sels métalliques, les dérivés acides polyméthacryliques (PMAA), les dérivés polyvinyloxazolidones (PVO), les dérivés polyvinylméthyloxazolidones (PVMO), les dérivés polyvinyloxazolines.

Comme autres agents intercalants polymériques hydrophiles, on peut citer également les copolymères des motifs cités ci-dessus, copolymères entre ces mêmes motifs ou copolymères avec d'autres monomères hydrophiles ou fortement polaires tels que : (méth)acrylate d'hydroxyéthyle, (méth)acrylate de 2 hydroxypropyle, (méth)acrylate de méthyle, acétate de vinyle, (méth)acrylamide, NN diméthyl acrylamide, acide crotonique, anhydride maléique, méthyl vinyl éther.

Les agents intercalants polymériques peuvent également être organophiles ou même lipophiles à condition de posséder au moins un, voire plusieurs groupe(s) polaire(s), tel(s) que défini(s) précédemment. On retiendra en particulier les copolymères obtenus par réaction entre un monomère polaire et hydrophile tel que choisi ci-dessus (vinyl pyrrolidone, vinyl oxazoline, vinyl oxazolidone, alcool vinylique, acide (méth)acrylique) avec au moins un monomère plus organophile voire lipophile tel que les esters d'acide (méth)acrylique tels (méth)acrylates d'éthyle, de butyle, d'isobutyle, de tertiobutyle, d'hexyle, de cyclohexyle, d'octyle, de 2-éthylhexyle, nonyle, décyle, undécyle, dodécyle, ... octadécyle, béhényle, les esters vinyliques tels que propianate, versatate, benzoate ; les (méth)acrylamides tels que diacétone acrylamide, butyl (méth)acrylamide, les tertiobutyl (méth)acrylamide, tertiohexyl (méth)acrylamide, tertiooctyl (méth)acrylamide ; les oléfines telles que éthylène, propylène, butène, isobutène, hexène, octène, dodecène, octadécène, eicosène, le styrène et les styrènes substitués.

D'autres polyols polymériques hydrosolubles et alcools polyhydriques, hydrosolubles ou hydrophiles tels que les polysaccharides sont également susceptibles de constituer des agents intercalants polymériques.

Certaines associations phyllosilicates/agent intercalant telles que montmorillonite-PVP, montmorillonite-PVA, et montmorillonite-alkylpyrrolidone comme par exemple la montmorillonite-dodécylpyrrolidone et leurs mélanges sont particulièrement avantageuses dans le cadre de la présente invention.

L'intercalation des phyllosilicates, considérés dans le cadre de la présente invention, peut être réalisée selon des protocoles classiques tels que ceux notamment décrits dans le brevet US 5 721 306 et le document WO 93/04118.

En l'occurrence, les agents intercalants peuvent être introduits ou adsorbés à l'intérieur des espaces interfoliaires du phyllosilicate selon le mode de réalisation suivant : l'intercalation est réalisée en mélangeant intimement les phyllosilicates par extrusion ou agitation à hélice en assurant, de préférence, un cisaillement important de manière à former une composition intercalante comprenant le phyllosilicate dans un polymère intercalant, une solution aqueuse d'agent intercalant ou une solution organique d'agent intercalant. Pour obtenir une intercalation suffisante en vue de l'exfoliation, l'agent intercalant est généralement mis en présence du phyllosilicate dans la composition d'intercalation à raison d'un rapport pondéral agent intercalant/phyllosilicate d'au moins environ 1/20, notamment d'au moins environ 1/10, plus particulièrement d'environ 1/2 à 1/5, voire d'environ 1/4, de manière à obtenir une intercalation efficace de l'agent entre des feuillets adjacents de phyllosilicate. L'espace interfoliaire peut être ainsi augmenté de 10 à 100 angstroms pour garantir une exfoliation consécutive, facile et totale. Le véhicule d'intercalation, de préférence l'eau, le cas échéant en mélange avec un solvant organique, peut être introduit après pré-solubilisation ou pré-dispersion de l'agent intercalant dans le véhicule, ou être directement mélangé avec l'agent intercalant sec et le phyllosilicate sec. L'agent intercalant est généralement présent en proportion à raison d'au moins 15 % en poids, notamment d'au moins 20 % en poids et plus particulièrement d'au moins 30 % en poids par rapport au poids de phyllosilicate sec (comprenant moins de 5 % d'eau). Cette quantité peut notamment varier de 20 à 50 % en poids par rapport au poids de phyllosilicate à l'état sec.

Les agents intercalants polymères peuvent souvent être obtenus par polymérisation (homo ou copolymérisation) directe des monomère(s) préalablement intercalés entre les feuillets du phyllosilicate.

En ce qui concerne l'exfoliation des phyllosilicates ainsi intercalés, elle est réalisée de manière conventionnelle, généralement par application au milieu dans lequel sont dispersés les phyllosilicates intercalés, une vitesse de cisaillement suffisante pour provoquer la délamination recherchée. Cet aspect est plus particulièrement développé ci-après.

Il existe par ailleurs un certain nombre de phyllosilicates intercalés et exfoliés déjà disponibles commercialement.

A titre illustratif des phyllosilicates exfoliés convenant à l'invention, on peut plus particulièrement citer ceux commercialisés par :
a) la société NANOCOR aux Etats-Unis. Comme exemples d'argiles (type montmorillonite) intercalées par un alkylammonium quaternaire, c'est-à-dire par échange d'ions, on peut citer les NANOMER 1.24 T, I.30 TC et I.34 TCN ; comme exemples d'argiles intercalées par un dérivé organique de pyrrolidone (technologie ion-dipole par échange de l'eau coordonnée) les NANOMER 1.35 K et 1.46 D, également décrits dans : « Advances in monomer additives for clay/polymer nanocomposites » LAN, T. (NANOCOR), Additives 99, International Conference, 8th, San Francisco, March 22-24, 1999 (1999) Paper 12/1 - Paper 12/11, Publisher = Executive Conference Management, Plymouth, Mic.
b) la société SOUTHERN CLAY PRODUCTS aux Etats-Unis, qui produit également des argiles intercalées par un alkylammonium quaternaire ex CLOISITE 25A et CLOISITE 30B cités dans l'article de POITTEVIN B., Polymer 43, 4017-23 (2002).

Les phyllosilicates exfoliés selon l'invention sont présents en une quantité efficace pour procurer à la composition cosmétique de bonnes propriétés en terme de tenue et notamment de non transfert et/ou non migration ou encore pour gélifier la phase grasse et/ou la phase organique de ladite composition.

En particulier, les phyllosilicates exfoliés peuvent être présents à raison de 0,05 à 20 % en poids, notamment de 0,1 à 15 % en poids et plus particulièrement de 0,5 à 10 % en poids dans la composition cosmétique.

Dans le cas où les argiles exfoliées sont mises en oeuvre à des fins de stabilisation d'émulsion, la quantité efficace est, pour des raisons évidentes, susceptible de varier significativement selon que ces argiles sont associées ou non à d'autre(s) tensioactif(s). L'homme de l'art est à même de procéder aux ajustements correspondants.

Ces phyllosilicates sont généralement directement introduits sous une forme exfoliée dans la composition. Toutefois, l'invention s'étend également aux compositions dans lesquelles les phyllosilicates exfoliés seraient *générés in situ,* par exemple par simple agitation de la composition cosmétique contenant des phyllosilicates intercalés c'est à dire sous une forme non exfoliée, voire du dispositif dans lequel est conditionnée ladite composition. Comme précisé précédemment, les phyllosilicates exfoliés selon l'invention peuvent être présents en mélange avec des phyllosilicates intercalés non exfoliés, de nature chimique identique ou non.

### MILIEU PHYSIOLOGIQUEMENT ACCEPTABLE :

Par « milieu physiologiquement acceptable », on désigne un milieu non toxique et susceptible d'être appliqué sur la peau ou les lèvres d'êtres humains. Le milieu physiologiquement acceptable est généralement adapté à la nature du support sur lequel doit être appliquée la composition ainsi qu'à l'aspect sous lequel la composition est destinée à être conditionnée.

### Phase grasse

La composition, notamment lorsqu'elle est destinée à être appliquée sur les lèvres ou sur la peau par exemple sous la forme d'un fond de teint, peut comporter notamment au moins un corps gras liquide à température ambiante (25°C) et à pression atmosphérique et/ou un corps gras solide à température ambiante et à pression atmosphérique tel que les cires, les corps gras pâteux, les gommes et leurs mélanges. La phase grasse peut en outre contenir des agents gélifiants et structurants d'huiles de nature organique et/ou des solvants organiques lipophiles.

En l'occurrence, la composition selon l'invention comprend au moins une phase grasse, liquide.

En particulier, la phase grasse peut constituer la phase continue ou la phase dispersée d'une émulsion, multiple ou non, et notamment d'une microémulsion.

En particulier, la composition peut posséder par exemple une phase grasse continue, pouvant contenir moins de 10 % en poids d'eau, notamment moins de 5 % en poids d'eau, voire moins de 1 % en poids d'eau par rapport à son poids total, et en particulier peut être sous forme anhydre.

Selon une autre variante particulière de l'invention, la composition contient moins de 70 % en poids, notamment moins de 50 % en poids, en particulière moins de 20 % en poids d'une phase aqueuse, par rapport au poids total de la composition. Au sens de l'invention, le terme « phase aqueuse » désigne une phase constituée d'un ou plusieurs solvant(s) hydrophile(s) tel(s) que l'eau, les glycols, la glycérine, les alcools et leurs mélanges.

En particulier, la phase grasse liquide peut constituer la phase continue ou la phase dispersée d'une émulsion.

Par phase grasse liquide structurée, on entend une phase grasse liquide rigidifiée ou gélifiée ou seulement épaissie.

Par phase grasse liquide gélifiée ou épaissie, on entend que la viscosité de cette phase grasse est augmentée du fait de son association avec un phyllosilicate exfolié selon l'invention.

La phase grasse de la composition selon l'invention peut notamment comprendre, à titre de corps gras liquide, au moins une huile volatile ou non volatile ou un de leurs mélanges.

La phase huileuse de la composition selon l'invention peut être présente en proportion variant de 1 à 80 %, en particulier de 1 à 50 % en poids, par rapport au poids total de la composition.

Par « huile volatile », on entend au sens de l'invention toute huile susceptible de s'évaporer au contact de la peau en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,01 à 300 mm de Hg (1,33 Pa à 40.000 Pa) et de préférence supérieure à 0,3 mm de Hg (30 Pa).

Par "huile non volatile", on entend une huile restant sur la peau à température ambiante et pression atmosphérique au moins plusieurs heures et ayant notamment une pression de vapeur inférieure à 0,01 mm de Hg (1,33 Pa).

Ces huiles volatiles ou non volatiles peuvent être des huiles hydrocarbonées notamment d'origine animales ou végétales, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permetyls® , les esters ramifiés en C₈-C₁₆ tels que le néopentanoate d'iso-hexyle, et leurs mélanges. D'autres huiles hydrocarbonées volatiles comme les distillats de pétrole, notamment ceux vendus sous la dénomination Shell Solt® par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 8 centistokes (8 x 10⁻⁶ m²/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile peut être présente dans la composition selon l'invention à une teneur allant de 0,1 % à 98 % en poids, notamment de 1% à 65 % en poids, et en particulier de 2 % à 50 % en poids, par rapport au poids total de la composition.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment les huiles de germe de blé, de tournesol, de pépins de raisin, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810® , 812® et 818® par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane et leurs mélanges,
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le palmitate de 2-éthyl-hexyle, l'isostéarate d'isostéaryle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle ; les esters de polyols et les esters du pentaérythritol,
- les alcools gras liquides à température ambiante à chaîne carbonée ramifiée et/ou insaturée ayant de 12 à 26 atomes de carbone comme l'octyldodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique,
- les huiles siliconées de type polyméthylsiloxane (PDMS) et leurs mélanges.

Plus précisément, les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates.

Les huiles non volatiles peuvent être présentes dans la composition selon l'invention en une teneur allant de 0,01 à 90 % en poids, notamment de 0,1 % à 85% en poids, et en particulier de 1 % à 70 % en poids, par rapport au poids total de la composition.

Plus généralement, le corps gras liquide à température ambiante et à pression atmosphérique peut être présent à raison de 0,01 à 90 % en poids et notamment de 0,1 à 85 % en poids, par rapport au poids de la phase grasse.

En ce qui concerne le corps gras solide à température ambiante et à pression atmosphérique, il peut être choisi parmi les cires, les corps gras pâteux, les gommes et leurs mélanges. Ce corps gras solide peut être présent à raison de 0,01 à 50 %, notamment de 0,1 à 40 % et en particulier de 0,2 à 30 % en poids, par rapport au poids total de la phase grasse.

Ainsi, la composition selon l'invention peut comprendre au moins un composé gras pâteux à température ambiante.

Par "pâteux" au sens de la présente invention, on entend un composé gras lipophile à changement d'état solide/liquide réversible, présentant à l'état solide une organisation cristalline anisotrope, et comportant à la température de 23 °C une fraction liquide et une fraction solide.

Par composé pâteux au sens de l'invention, on entend un composé ayant une dureté à 20°C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20°C au centre de 5 échantillons. Le cylindre est introduit dans chaque échantillon à une pré-vitesse de 1mm/s puis à une vitesse de mesure de 0,1 mm/s, la profondeur de pénétration étant de 0,3 mm. La valeur relevée de la dureté est celle du pic maximum.

Ce composé pâteux est en outre, à la température de 23°C, sous la forme d'une fraction liquide et d'une fraction solide. En d'autres termes, la température de fusion commençante du composé pâteux est inférieure à 23°C. La fraction liquide du composé pâteux mesurée à 23°C représente de 9 à 97% en poids du composé. Cette fraction liquide à 23°C représente de préférence de 15 à 85%, de préférence encore de 40 à 85% en poids par rapport au poids total du composé.

La fraction liquide en poids du composé pâteux à 23°C est égale au rapport de l'enthalpie de fusion consommée à 23°C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide cristalline. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée en J/g.

L'enthalpie de fusion consommée à 23°C est la quantité d'énergie absorbée par l'échantillon pour passer de l'état solide à l'état qu'il présente à 23°C, c'est-à-dire constitué d'une fraction liquide et d'une fraction solide.

La fraction liquide du composé pâteux mesurée à 32 °C représente de préférence de 30 à 100 % en poids du composé, de préférence de 80 à 100 %, de préférence encore de 90 à 100 % en poids du composé. Lorsque la fraction liquide du composé pâteux mesurée à 32 °C est égale à 100 %, la température de la fin de la plage de fusion du composé pâteux est inférieure ou égale à 32 °C.

La fraction liquide du composé pâteux mesurée à 32 °C est égale au rapport de l'enthalpie de fusion consommée à 32 °C sur l'enthalpie de fusion du composé pâteux. L'enthalpie de fusion consommée à 32 °C est calculée de la même façon que l'enthalpie de fusion consommée à 23 °C.

Plus particulièrement, ces corps gras peuvent être des composés hydrocarbonés, éventuellement de type polymérique ; ils peuvent également être choisis parmi les composés siliconés; ils peuvent aussi se présenter sous forme d'un mélange de composés hydrocarbonés et/ou siliconés. Dans le cas d'un mélange de différents corps gras pâteux, on utilise de préférence les composés pâteux hydrocarbonés (contenant principalement des atomes de carbone et d'hydrogène et éventuellement des groupements ester), en proportion majoritaire.

Parmi les composés pâteux susceptibles d'être utilisés dans la composition selon l'invention, on peut citer les lanolines et les dérivés de lanoline comme les lanolines acétylées, les lanolines oxypropylènées ou le lanolate d'isopropyle, et leurs mélanges. On peut également utiliser des esters d'acides ou d'alcools gras, notamment ceux ayant 20 à 65 atomes de carbone comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux et leurs mélanges. Comme triglycéride d'origine végétale, on peut utiliser les dérivés d'huile de ricin hydrogénée, tels que le « THIXINR® » de Rheox.

On peut aussi citer les corps gras pâteux siliconés tels que les polydiméthylsiloxanes (PDMS) de hauts poids moléculaires et en particulier ceux ayant des chaînes pendantes du type alkyle ou alcoxy ayant de 8 à 24 atomes de carbone, et un point de fusion de 20-55°C, comme les stéaryl diméthicones notamment ceux vendus par la société Dow Corning sous les noms commerciaux de DC2503® et DC25514® et leurs mélanges.

Le corps gras pâteux peut être présent dans la composition selon l'invention en une teneur allant de 0,01 à 50% en poids, notamment allant de 0,1 à 45 % en poids, et en particulier allant de 0,2 % à 30 % en poids, par rapport au poids total de la composition.

La composition selon l'invention peut comprendre en outre une cire. La cire peut être solide à température ambiante (25°C), à changement d'état solide/liquide réversible, ayant une température de fusion supérieure à 30°C pouvant aller jusqu'à 200°C, une dureté supérieure à 0,5MPa et présentant à l'état solide une organisation cristalline anisotrope. Elle peut être hydrocarbonée, fluorée et/ou siliconée et être d'origine animale, végétale, minérale ou synthétique. Elle peut être choisie par exemple parmi la cire d'abeille, la cire de Carnauba, la cire de Candellila, les cires de paraffine, l'huile de ricin hydrogénée, les cires de silicone, et les cires microcristallines et leurs mélanges.

En particulier, la cire peut être présente sous forme d'émulsion cire(s)-dans-eau.

La cire peut être présente dans la composition selon l'invention en une teneur allant de 0,01 % à 50 % en poids, en particulier de 0,1 % à 30 % en poids, et notamment de 0,2 % à 20 % en poids, par rapport au poids total de la composition.

### Emulsion :

Au sens de la présente invention, on entend par « émulsion », un système de deux liquides non miscibles dont l'un est finement divisé en gouttelettes dans l'autre. La phase dispersée est encore appelée « phase interne ou discontinue ». La phase dispersante est aussi appelée « phase externe ou continue ». Les émulsions dans lesquelles la phase dispersée est lipophile, huile végétale ou minérale par exemple, et la phase dispersante hydrophile, par exemple l'eau, sont dites de type aqueux (H/E : huile-dans-eau). Les émulsions dans lesquelles la phase dispersée est hydrophile et la phase dispersante lipophile sont dites de type huileux (E/H : eau-dans-huile). Il existe également des émulsions dites multiples, par exemple E/H/E : eau-dans-huile-dans-eau.

Ainsi, les émulsions contiennent une phase lipophile et une phase hydrophile, cette dernière n'étant pas systématiquement de l'eau.

En particulier, la composition peut être translucide ou transparente et/ou peut être susceptible de donner un dépôt translucide ou transparent.

Selon un mode de réalisation, la composition contient sous forme d'émulsion au moins un agent émulsionnant et éventuellement au moins un agent co-émulsionnant en une proportion inférieure à 30%, de préférence inférieure à 20 %, de préférence inférieure à 10 %. Selon un mode de réalisation, la composition sous forme d'émulsion contient au moins un agent émulsionnant et éventuellement au moins un agent co-émulsionnant en une proportion allant de 0,2 à 30 % en poids, de préférence de 0,3 à 20 % en poids et mieux de 0,5 à 15 % en poids par rapport au poids total de la composition.

De manière plus particulière, la composition contient moins de 0,5 % en poids par rapport au poids total de la composition d'agents émulsionnants et d'agents co-émulsionnants.

En particulier, la composition de l'invention sous forme d'une émulsion est avantageusement exempte de tout composé émulsionnant permettant de la stabiliser à l'exception du phyllosilicate exfolié conforme à l'invention.

L'homme du métier sait déterminer aisément, pour une composition comprenant une phase aqueuse et une phase grasse déterminées, dans un ratio déterminé, le type d'émulsionnant et éventuellement de co-émulsionnant de l'art antérieur ainsi que leurs proportions respectives permettant d'obtenir une émulsion stable. Par émulsion stable, on entend une émulsion qui, lorsqu'elle est placée dans un récipient transparent, lui-même placé dans une étuve à 45°C pendant deux mois, ne déphase pas (ou n'exsude pas) au bout de cette période. Le déphasage (ou exsudation) est détecté à l'oeil nu dès la sortie du récipient de l'étuve à travers les parois transparentes.

La composition peut notamment se présenter sous forme d'émulsions comportant une phase aqueuse et une phase huileuse dispersées l'une dans l'autre, par exemple sous forme d'émulsions eau-dans-huile (E/H) ou huile-dans-eau (H/E) ou multiple (E/H/E ou H/E/H), choisies notamment parmi les émulsions habituelles ou les émulsions particulières comme par exemple parmi :
- les émulsions H/E à base de globules huileux pourvus d'un enrobage cristal liquide lamellaire, telles que décrites dans les documents EP-A-641557 et EP-A-705593 ;
- Les émulsions H/E sans tensioactif, stabilisées par des polymères anioniques hydrodispersibles, telles que celles décrites dans le document EP-A-864320 ;
- Les émulsions H/E à base de polymères dérivés d'acide 2-acrylamido 2-méthylpropane sulfonique (Polymère AMPS), telles que décrites dans le document EP-A-815844 ;
- Les émulsions H/E stabilisées par des polymères d'AMPS hydrophobes, telles décrites dans les documents EP-A-1,069,142, WO-A-2002/43689, WO-A-2002/44231, WO-A-2002/44271, WO-A-2002/44270, WO-A-2002/43686, WO-A-2002/44267, WO-A-2002/43688, WO-A-2002/43677, WO-A-2002/43687, WO-A-2002/44230 ;
- Les émulsions fluides à base de polymères thermo associatifs, telles que décrites dans les documents EP-A-1,355,990, EP-A-1,355,625, EP-A-1,307,501, EP-A-1,363,964 ;
- Les émulsions H/E obtenues par la méthode PIT (émulsion obtenue par inversion de phase, PIT : Phase Inversion Temperature), telles que décrites dans les documents WO-A-89/11907, DE-A-4318171, et EP-A-815846 ;
- Les nanoémulsions telles que celles décrites dans les demandes EP-A-728460, EP-A-780114, EP-A-780115, EP-A-879589, EP-A-1,010,413, EP-A-1,010,414, EP-A-1,010,415, EP-A-1,010,416, EP-A-1,013,338, EP-A-1,016,453, EP-A-1,018,363, EP-A-1,020,219, EP-A-1,025,898, EP-A-1,120,102, EP-A-1,120,101, EP-A-1,160,005, EP-A-1,172,077 et EP-A-1,353,629.

La proportion de la phase huileuse de l'émulsion peut varier de 1 à 80 % en poids, et de préférence de 1 à 50 % en poids par rapport au poids total de la composition. Les huiles, et les agents émulsionnants et co-émulsionnants éventuellement présents, utilisés dans la composition sous forme d'émulsion, sont choisis parmi ceux généralement utilisés dans le domaine cosmétique ou dermatologique. L'agent émulsionnant et l'agent co-émulsionnant, quand ils sont présents, sont généralement en proportion variant de 0,2 à 30 % en poids, de préférence de 0,3 à 20 % en poids et mieux de 0,5 à 15 % en poids par rapport au poids total de la composition. L'émulsion peut, en outre, contenir des vésicules lipidiques.

L'agent émulsionnant peut être choisi parmi les émulsionnants amphotères, anioniques, cationiques ou non ioniques, utilisés seuls ou en mélange. Les agents émulsionnants sont choisis de manière appropriée, suivant la phase continue de l'émulsion à obtenir (E/H ou H/E). Quand l'émulsion est multiple, elle comporte généralement un agent émulsionnant dans l'émulsion primaire et un émulsionnant dans la phase externe dans laquelle est introduite l'émulsion primaire.

Comme agents émulsionnants utilisables pour la préparation des émulsions E/H, on peut également citer les alkyl esters ou éthers de sorbitan, de glycérol ou de sucres ; les tensioactifs siliconés comme les dimethicone copolyols tels que le mélange de cyclomethicone et de dimethicone copolyol, vendu sous les dénominations DC 5225 C et DC 3225 C par la société Dow Corning, et comme les alkyl-dimethicone copolyols tels que le Laurylmethicone copolyol vendu sous la dénomination "Dow Corning 5200 Formulation Aid" par la société Dow Corning, le Cetyl dimethicone copolyol vendu sous la dénomination Abil EM 90^{R} par la société Goldschmidt et le mélange de Polyglyceryl-4 isostearate/Cetyl dimethicone copolyol/Hexyl laurate vendu sous la dénomination Abil WE 09^{R} par la société Goldschmidt. On peut y ajouter aussi un ou plusieurs co-émulsionnants, qui, de manière avantageuse, peuvent être choisis dans le groupe comprenant les esters d'acide gras à chaîne ramifiée et de polyol, et notamment les esters d'acide gras à chaîne ramifiée et de glycérol et/ou de sorbitan et par exemple l'isostéarate de polyglycéryle, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Comme agents émulsionnants utilisables pour la préparation des émulsions H/E, on peut citer par exemple les agents émulsionnants non ioniques tels que les esters d'acides gras et de polyols oxyalkylénés (plus particulièrement polyoxyéthylénés), et par exemple les stéarates de polyéthylène glycol comme le stéarate de PEG-100; le stéarate de PEG-50 et le stéarate de PEG-40 ; et leurs mélanges tels que le mélange de mono-stéarate de glycéryle et de stéarate de polyéthylène glycol (100 OE) commercialisé sous la dénomination SIMULSOL 165 par la société SEPPIC ; les esters d'acides gras et de sorbitan oxyalkylénés comprenant par exemple de 20 à 100 OE, et par exemple ceux commercialisés sous les dénominations commerciales Tween 20 ou Tween 60 par la société Ubiqema ; les éthers d'alcools gras oxyalkylénés (oxyéthylénés et/ou oxypropylénés) ; les esters de sucres, alcoxylé ou non, comme le stéarate de sucrose et comme le PEG-20 méthylglucose sesquistéarate ; les esters de sorbitan tels que le palmitate de sorbitan commercialisé sous la dénomination Span 40 par la société Uniqema ; les esters de diacide et d'alcool gras, tels que le tartrate de dimyristyle ; les mélanges de ces émulsionnants comme par exemple le mélange de stéarate de glycéryle et de stéarate de PEG-100, commercialisé sous la dénomination Arlacel 165 par la société Uniqema ; et les mélanges contenant ces agents émulsionnants, tels que le mélange de tartrate de dimyristyle, d'alcool cétéarylique, de Pareth-7 et de PEG-25 laureth-25, commercialisé sous la dénomination Cosmacol PSE par la société Sasol (nom CTFA : Dimyristyl tartrate / cetearyl alcool /12-15 Pareth 7 / PPG 25 laureth 25).

On peut ajouter à ces agents émulsionnants, des agents co-émulsionnants tels que par exemple les alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique, ou les acides gras.

On peut aussi préparer des émulsions sans tensioactifs émulsionnants ou en contenant moins de 0,5 % du poids total de la composition, en utilisant des composés appropriés, par exemple les polymères ayant des propriétés émulsionnantes tels que les polymères commercialisés sous les dénominations CARBOPOL 1342 et PEMULEN par la société Noveon ; ou les polymères en émulsion tels que celui commercialisé sous la dénomination Sepigel 305 par la société Seppic (nom INCI : Polyacrylamide / C13-C14 isoparaffine / laureth-7) ; les particules de polymères ioniques ou non ioniques, plus particulièrement des particules de polymère anionique comme notamment les polymères d'acide isophtalique ou d'acide sulfoisophtalique, et en particulier les copolymères de phtalate / sulfoisophtalate / glycol (par exemple diéthylèneglycol / Phtalate /isophtalate/1,4-cyclohexane-diméthanol (nom INCI : Diglycol/CHDM/Isophtalates/ SIP Copolymer) vendus sous les dénominations Eastman AQ polymer (AQ35S, AQ38S, AQ55S, AQ48 Ultra) par la société Eastman Chemical. On peut aussi préparer des émulsions sans émulsionnants, stabilisées par des particules siliconées ou des particules d'oxyde métallique tels que TiO2 ou autres.

### Polymères filmogènes :

La composition selon l'invention peut comprendre, en outre, au moins un polymère filmogène.

Selon la présente invention, on entend par "polymère filmogène", un polymère apte à former à lui seul ou en présence d'un agent auxiliaire de filmification, un film continu à l'oeil nu sur un support.

Le polymère filmogène peut être organique ou inorganique. Dans une mise en oeuvre particulière de l'invention, le polymère filmogène organique est au moins un polymère choisi parmi le groupe comprenant :
- les polymères filmogènes liposolubles, et
- les polymères filmogènes lipodispersibles sous forme de dispersions non aqueuses de particules de polymère, de préférence des dispersions de particules polymériques, le cas échéant stabilisées en leur surface par au moins un agent stabilisant, dans une ou plusieurs huiles de silicones et/ou hydrocarbonées ; ces dispersions non aqueuses sont encore appelées « NAD ».

### 1. Polymères liposolubles

Les polymères liposolubles peuvent être de toute nature chimique et englobent notamment :
a) les homopolymères et les copolymères liposolubles et amorphes d'oléfines, de cyclo-oléfines, de butadiene, d'isoprène, de styrène, d'éthers, d'esters ou d'amides vinyliques, d'esters ou d'amides d'acide (méth)acrylique comprenant un groupement alkyl en C₄₋₅₀ linéaire, ramifié ou cyclique, et de préférence amorphe.
   Comme copolymères liposolubles particuliers, on peut citer :
   i) les polymères greffés acrylique-silicone à squelette silicone, greffons acryliques ou à squelette acrylique, greffons silicones tels que le produit vendu sous la dénomination SA 70.5 par 3M et ceux décrits dans les brevets US 5 725 882, US 5 209 924, US 4 972 037, US 4 981 903, US 4 981 902, US 5 468 477, US 5 219 560 et EP 0 388 582.
   ii) les polymères liposolubles porteurs de groupes fluorés appartenant à l'une des classes décrites ci-dessus, en particulier ceux décrits dans le brevet US 5 948 393, les copolymères (méth)acrylate d'alkyle / (méth)acrylate de perfluoroalkyle décrits dans les brevets EP 0 815 836 et US 5 849 318.
   iii) les polymères ou copolymères résultant de la polymérisation ou de la copolymérisation d'un monomère éthylénique, comprenant un ou plusieurs motifs éthyléniques, de préférence conjugués (ou diènes). Comme polymères ou copolymères résultant de la polymérisation ou de la copolymérisation d'un monomère éthylénique, on peut utiliser les copolymères vinyliques, acryliques ou méthacryliques qui peuvent être des copolymères en blocs, tels que les copolymères di-blocs ou tri-blocs, ou même des copolymères multiblocs à formes variées. L'agent filmogène, comprenant au moins un motif éthylénique, peut comprendre, par exemple, un bloc styrène (S), un bloc alkylstyrène (AS), un bloc éthylène/butylène (EB), un bloc éthylène/propylène (EP), un bloc butadiène (B), un bloc isoprène (I), un bloc acrylate (A), un bloc méthacrylate (MA) ou une combinaison de ces blocs.
   iv) les copolymères de N-vinyl pyrrolidone et d'oléfines, oléfines dont le nombre d'atomes de carbone est > à 8 ex copolymère N-vinyl pyrrolidone/hexadécène, N-vinyl pyrrolidone/eicosène.
   v) les homo ou copolymères d'esters vinyliques liposolubles comme les polylaurates de vinyle, polystéarate de vinyle et leurs copolymères avec l'acétate de vinyle.
   vi) les homo ou copolymères d'esters ou d'amides(méth)acryliques. Les monomères esters méthacryliques résultent alors de l'estérification de l'acide (meth)acrylique avec un alcool ayant un nombre d'atomes de carbone > 4 et de préférence ≥ à 8 avec comme exemple (méth)acrylate 2 éthylhexyle, (méth)acrylate de n-octyle, (méth)acrylate de dodécyle, (méth)acrylate de stéaryle, (méth)acrylate de béhényle. Ces (méth)acrylates à longue chaîne peuvent être copolymérisés avec d'autres esters (méth)acryliques, esters vinyliques ou styrène.
b) les polycondensats liposolubles, amorphes, notamment ne comportant pas de groupes donneurs de liaisons hydrogène, en particulier les polyesters à chaînes latérales alkyles en C₄₋₅₀, les polyesters résultant de la condensation d'acides gras dimères, ou les polyesters comportant un segment silicone sous forme de séquence, greffon, ou groupe terminal, solides à température ambiante,
c) les polysaccharides liposolubles et amorphes comportant des chaînes latérales alkyles (éthers ou esters), en particulier les alkylcelluloses portant des radicaux alkyles linéaires ou ramifiés, saturés ou insaturés en C₁ à C₈, tels que l'éthylcellulose et le propylcellulose.

D'une façon générale, les polymères liposolubles filmogènes susceptibles d'être mis en oeuvre selon l'invention peuvent avoir un poids moléculaire compris entre 1.000 et 500.000, de préférence entre 2.000 et 250.000, et une température de transition vitreuse comprise entre -100 °C et +300 °C, notamment entre -50 °C et +100 °C, et en particulier entre -10 °C et + 90 °C.

### 2. Polymères lipodispersibles : dispersions non aqueuses (NAD) de particules de polymères

Il s'agit de dispersions non aqueuses stables de particules de polymères, généralement sphériques, d'un ou plusieurs polymères, dans une phase grasse liquide physiologiquement acceptable, tels que les huiles hydrocarbonées ou les huiles de silicone. Ces dispersions sont généralement appelées dispersions non aqueuses (NAD) de polymères. Ces dispersions peuvent en particulier se présenter sous forme de dispersions stables de nanoparticules de polymères dans ladite phase grasse. Selon une variante particulière, la taille de ces nanoparticules varie entre 5 nm et 600 nm. Cependant, il est possible d'obtenir des particules de polymères allant jusqu'à 1 µm.

Les dispersions de polymère de ce type offrent avantageusement la possibilité de faire varier la température de transition vitreuse (Tg) du polymère ou du système de polymère (polymère plus additif de type plastifiant), et donc d'évoluer d'un polymère dur vers un polymère plus ou moins mou. Il est ainsi possible d'ajuster les propriétés mécaniques de la composition en fonction de l'application visée, par exemple au niveau du film déposé.

Les polymères en dispersion utilisables dans la composition selon l'invention ont de préférence un poids moléculaire d'environ 2 000 à 10 000 000 et une Tg de -100 °C à 300 °C, en particulier de -50 °C à 50 °C, et notamment de -10 °C à 100 °C.

Il est possible d'utiliser des polymères filmogènes qui ont de préférence une Tg basse, inférieure ou égale à la température de la peau et en particulier inférieure ou égale à 40 °C. La dispersion ainsi obtenue peut former un film lorsqu'elle est appliquée sur un support.

Parmi les polymères filmogènes qui peuvent être cités, figurent tout particulièrement les homopolymères ou les copolymères de vinyle ou d'acrylique de type radicalaire, ayant notamment une Tg inférieure ou égale à 40 °C et plus particulièrement variant de -10 °C à 30 °C, utilisés seuls ou en mélange.

L'expression « polymère de type radicalaire » couvre un polymère obtenu par polymérisation de monomères contenant une insaturation, plus particulièrement une insaturation éthylénique ; chaque monomère étant capable d'homopolymérisation (à la différence des polycondensats). Ces polymères de type radicalaire peuvent plus particulièrement être des polymères ou des copolymères vinyliques, en particulier des polymères acryliques.

Les polymères de type radicalaire préférentiellement utilisés sont les copolymères d'acide (méth)acrylique et d'un (méth)acrylate d'alkyle, plus particulièrement d'un alkyle en C₁-C₄. Préférentiellement, les acrylates de méthyle peuvent être utilisés, éventuellement copolymérisés avec un acide acrylique.

D'une manière non limitative, les polymères en dispersion selon l'invention peuvent être choisis parmi les polymères ou copolymères suivants : les polyuréthanes, les polyuréthanes acryliques, les polyurées, les polyuréthanes de polyurée, les polyuréthanes de polyester, les polyuréthanes de polyéther, les polyesters, les polyester-amides, les polyesters à chaîne grasse, les polymères ou copolymères vinyliques et/ou acryliques, les copolymères de silicone/acrylique, les polyacrylamides, les polymères de silicone, par exemple les polyuréthanes de silicone ou les silicones d'acryliques, les fluoro-polymères et leurs mélanges.

Le ou les polymère(s) en dispersion huileuse peuvent représenter (en tant que matière active ou solide) de 0,1 % à 60 % en poids de la composition, notamment de 2 % à 40 % et encore mieux de 4 % à 25 %. Dans le cas de la présence d'un agent stabilisant solide à température ambiante, la teneur en solide en dispersion représente le nombre total de polymère et d'agent stabilisant.

Les polymères liposolubles ou lipodispersibles dans la composition selon l'invention peuvent être également utilisés en une quantité variant de 0,01 % à 40 % par rapport au poids total de la composition, notamment de 1 à 20 %, tel que, par exemple, de 1 % à 10 %.

Le polymère filmogène peut être associé à des agents auxiliaires de filmification. Un tel agent de filmification peut être choisi parmi tous les composés connus de l'homme du métier comme étant susceptibles de remplir la fonction recherchée, et notamment être choisi parmi les agents plastifiants et les agents de coalescence.

### Phase particulaire :

La composition de l'invention, peut en outre comprendre une phase particulaire additionnelle pouvant être présente à raison de 0,01 à 40 % en poids, notamment de 0,01 à 30 % en poids et en particulier de 0,05 à 20 % en poids, par rapport au poids total de la composition.

Elle peut notamment comprendre des pigments et/ou des nacres et/ou des charges complémentaires utilisés dans les compositions cosmétiques.

Par pigments, il faut comprendre des particules blanches ou colorées, minérales ou organiques, insolubles dans la phase hydrophile liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées.

Les pigments peuvent être présents dans la composition à raison de 0,01 à 25 % en poids, en particulier de 0,05 à 20 % en poids, et notamment de 0,1 à 15 % et en particulier de 0,5 à 10 % en poids par rapport au poids de la composition.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome. Parmi les pigments organiques utilisables dans l'invention, on peut citer le noir de carbone, les pigments de type D & C, et les laques à base de carmin de cochenille, de baryum, strontium, calcium, aluminium ou encore les dicétopyrrolopyrrole (DPP) décrits dans les documents EP-A-542669, EP-A-787730, EP-A-787731 et WO-A- 96/08537. La quantité et/ou le choix de ces pigments sont généralement ajustés en prenant en compte la quantité en phyllosilicates intercalés exfoliés présente dans la composition cosmétique considérée.

Les nacres peuvent être présentes dans la composition à raison de 0,01 à 25 % en poids, notamment de 0,01 à 15 % en poids, et en particulier de 0,02 à 5 % en poids, par rapport au poids total de la composition.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges complémentaires peuvent être présentes à raison de 0, 01 à 40 % en poids, notamment 0,01 à 30 % en poids, et en particulier de 0,02 à 20 % en poids par rapport au poids total de la composition. Leur quantité est également généralement ajustée en prenant en compte la quantité en phyllosilicates intercalés exfoliés.

Il peut notamment s'agir de charges sphériques comme par exemple le talc, le stéarate de zinc, le mica, le kaolin, les poudres de polyamide (Nylon® ) (Orgasol® de chez Atochem), les poudres de polyéthylène, les poudres de polymères de tétrafluoroéthylène (Téflon® ), l'amidon, le nitrure de bore, des microsphères polymériques telles que celles de chlorure de polyvinylidène/acrylonitrile comme l'Expancel® (Nobel Industrie), de copolymères d'acide acrylique (Polytrap® de la société Dow Corning), les microbilles de résine de silicone (Tospearls® de Toshiba, par exemple), et les organopolysiloxanes élastomères.

La composition peut comprendre également des colorants hydrosolubles ou liposolubles en une teneur allant de 0,01 à 6 % en poids, par rapport au poids total de la composition, notamment allant de 0,01 à 3 % en poids. Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, et le jaune quinoléine. Les colorants hydrosolubles sont par exemple le jus de betterave et le bleu de méthylène.

La composition selon l'invention peut, de plus, comprendre tous les ingrédients classiquement utilisés dans les domaines concernés et plus spécialement dans le domaine cosmétique et dermatologique. Ces ingrédients sont en particulier choisis parmi les vitamines, les antioxydants, les épaississants, les oligo-éléments, les adoucissants, les séquestrants, les parfums, les agents alcalinisants ou acidifiants, les conservateurs, les filtres UV, les actifs hydrophiles ou lipophiles et leurs mélanges. Les quantités de ces différents ingrédients sont celles classiquement utilisées dans les domaines concernés et par exemple de 0,01 à 20 % du poids total de la composition.

Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction considérée.

La composition de l'invention peut être obtenue selon les procédés de préparation classiquement utilisés en cosmétique, en dermatologie *et*/*ou soin des ongles.*

Selon un mode préféré de l'invention, on utilisera pour les formulations cosmétiques des argiles, de préférence montmorillonites, pré-intercalées que l'on va exfolier :
a) soit dans un pré-gel contenant au moins l'un des solvants de la formulation finale. Il peut s'agir par exemple d'un solvant volatil ou non volatil choisi parmi l'éthanol, un polyol tel que le propylène glycol, le glycérol, un hydrocarbure aliphatique volatil tel que l'isododécane, une huile hydrocarbonée polaire ou non polaire, une silicone volatile ou non volatile,
b) soit directement dans la formulation ou dans l'une des phases de la formulation (il s'agit par exemple d'une émulsion ou d'une dispersion), c'est-à-dire en présence des autres solvants et/ou huiles et/ou polymères et/ou pigments et charges.

Que ce soit selon le mode a) ou le mode b), l'exfoliation des phyllosilicates intercalés est réalisée par tous moyens susceptibles de conduire à une délamination d'au moins environ 80 % en poids dudit phyllosilicate. En l'occurrence, la vitesse de cisaillement requise pour réaliser ce type d'exfoliation peut nécessiter une vitesse de cisaillement d'au moins 10s⁻¹, voire plus. En l'occurrence, la limite supérieure de cette vitesse de cisaillement n'est pas critique.

Généralement, cette vitesse de cisaillement varie d'environ 10s⁻¹ à environ 20 000s⁻¹ et plus particulièrement d'environ 100s-¹ à environ 10 000s⁻¹. Dans certains cas, il peut être avantageux d'associer à ce cisaillement un chauffage et/ou une augmentation de la pression.

En ce qui concerne plus particulièrement le cisaillement en soit, il peut être assuré à l'aide de différents dispositifs conventionnels. Ce cisaillement peut notamment être réalisé à l'aide de moyens mécaniques, par choc thermique, altération de pression ou par ultrasons. Le choix du mode de cisaillement relève de la connaissance de l'homme de l'art.

Conviennent tout particulièrement à l'invention, les cisaillements obtenus par des modes mécaniques, comme notamment les agitateurs, homogénéisateurs ou disperseurs de type MORITZ® , ULTRATURRAX® , le mixeur de type BANBURY® ; les mélangeurs de type BRABENDER® et les extrudeurs, notamment de type KNEADER® .

Dans le cas particulier d'un cisaillement mécanique, notamment dans une extrudeuse, la température du milieu à exfolier, la longueur de l'extrudeuse, le temps de résidence de ce milieu dans l'extrudeuse et le type d'extrudeuse retenue, à savoir simple vis/double vis ..., sont autant de variables susceptibles de contrôler la force de cisaillement à appliquer pour l'exfoliation.

Une exfoliation est jugée suffisante lorsqu'elle procure au moins 80 % en poids, notamment au moins 85 %, en particulier au moins environ 90 % en poids, voire environ 95 % en poids de phyllosilicate exfolié.

Les phyllosilicates ainsi formés possèdent avantageusement une épaisseur variant de l'épaisseur de couches individuelles à l'épaisseur de une à cinq couches associées.

La composition peut se présenter sous diverses formes, en fonction de sa destination.

La composition cosmétique peut ainsi se présenter sous toute forme galénique normalement utilisée pour une application topique et notamment sous forme anhydre, sous forme d'une solution huileuse ou aqueuse, d'un gel huileux ou aqueux, d'une émulsion huile dans eau, eau dans huile, cire dans eau ou eau dans cire, d'une émulsion multiple, d'une dispersion d'huile dans de l'eau grâce à des vésicules situés à l'interface huile/eau, d'une émulsion directe ou inverse.

La composition peut se présenter sous forme de produit coulé, en coupelle ou sous forme de stick dans le cas d'un rouge à lèvres ou d'un produit de soin des lèvres.

La composition peut encore se présenter sous diverses autres formes, par exemple d'un liquide plus ou moins visqueux, d'un gel ou d'une pâte.

La composition peut encore se présenter sous la forme d'un semi-solide ou d'un solide, par exemple un pain à humidifier au moment de l'utilisation, de manière à lui permettre de se déliter.

Avantageusement, elle se présente sous forme de composition à phase continue huileuse pouvant comprendre moins de 10 % en poids d'eau, notamment moins de 5 % en poids d'eau, et en particulier anhydre.

La composition cosmétique peut constituer, entre autres, un rouge à lèvres, un baume à lèvres, un gloss liquide, une pâte de rouge à lèvres, un fard à joues, un vernis à ongles, un crayon à lèvres, un fond de teint solide, notamment coulé, ou fluide, de produits « correcteurs » ou « embellisseurs » de teint, un produit pour le soin et/ou le maquillage des ongles naturels ou synthétiques, un produit anti-cernes ou de contour des yeux, un eye-liner, un mascara, une ombre ou un fard à paupières, un produit de maquillage du corps ou des cheveux ou encore un produit solaire ou de coloration de la peau.

En particulier, il s'agit d'un vernis à ongles.

Les exemples figurant ci-après sont présentés à titre illustratif et non limitatif du domaine de l'invention.

### Exemple 1 et exemple comparatif: Préparation de vernis à ongles

On prépare un vernis selon l'invention en formulant des phyllosilicates constitués de montmorillonite modifiée par une amine grasse d'origine suif commercialisé sous la référence Nanomer 1.34TCN par Nanocor.

On prépare également selon le même procédé un vernis de l'art antérieur de même composition en remplaçant poids pour poids les phyllosilicates, par de l'hectorite modifiée par du chlorure de stéaryl diméthyl benzyl ammonium commercialisée sous la référence Bentone® 27V par Elementis.
a) On réalise un gel nitrocellulosique thixotrope en mélangeant à 25 °C sous une agitation appropriée
   - 13 g de nitrocellulose (agent filmogène)
   - 7 g de phyllosilicate intercalé ou d'hectorite
   - 0,3 g d'acide citrique (agent gonflant)
   - 8 g d'alcool isopropylique (solvant volatil)
   - qsp 100 g d'acétate de butyle
   100 parties du gel ainsi obtenu sont mélangées à 25°C à 450 parties d'une base incolore non thixotrope_ de composition comme suit :
   - 8 g de plastifiant
   - 24 g d'un mélange de nitrocellulose et d'un agent co-filmogène
   - 5 g d'alcool isopropylique
   - q.s.p 100 g d'acétate de butyle et acétate d'éthyle à 50/50

On obtient ainsi un vernis à ongle dont la composition est la suivante (pourcentages en poids) :
- agent filmogène (nitrocellulose, résine ...) 22 %
- plastifiant 6,5 %
- alcool isopropylique 5,5 %
- phyllosilicate ou hectorite 1,3 %
- acide citrique 0,05 %
- acétate de butyle 39,2 %
- acétate d'éthyle 26,1 %

La brillance de cette composition est mesurée à l'aide d'un brillancemètre, de manière conventionnelle par la méthode suivante.

Sur une carte de contraste de marque LENETA et de référence FORM 1A PENOPAC, on étale une couche d'environ 300 µm d'épaisseur de la composition à l'aide d'un étaleur automatique. La couche recouvre au moins le fond blanc de la carte. On laisse le dépôt sécher. On procède à la mesure de la brillance à 20° et à 60° sur le fond blanc à l'aide d'un brillancemètre de marque BYK GARDNER et de référence micro TRI-GLOSS. On obtient une valeur de la brillance moyenne comprise entre 0 et 100.

Les valeurs mesurées sont indiquées ci-après pour chacune des compositions testées.

| | Vernis transparent préparé avec la bentone B27 V commercialisée par Elementis | Vernis transparent préparé avec le phyllosilicate Nanomer I.34TCN commercialisé par Nanocor |
|---|---|---|
| Brillance à 20°/à 60° | 53/85 | 63/86 |

### Exemple 2 et exemple comparatif: Préparation d'un vernis à ongles

Dans un malaxeur bi-vis, on malaxe :
- 20 g d'hectorite ou de phyllosilicate tels que définis en exemple 1,
- 30 g d'acétobutyrate de cellulose d'Eastman Chemical CAB 3810.5,
- 50 g de n-éthyl-o,p-toluène-sulfonamide de PAN-AMERICANA (référence Resimpol 8).

5 parties du gel obtenu ci-dessus sont mélangées à 95 parties du gel nitrocellulosique thixotrope obtenu en exemple 1 *a).*

La brillance de cette composition est mesurée à l'aide d'un brillancemètre, de manière conventionnelle par la méthode suivante.

Sur une carte de contraste de marque LENETA et de référence FORM 1A PENOPAC, on étale une couche d'environ 300 µm d'épaisseur de la composition à l'aide d'un étaleur automatique. La couche recouvre au moins le fond blanc de la carte. On procède de suite à la mesure de la brillance à 20° et à 60° sur le fond blanc à l'aide d'un brillancemètre de marque BYK GARDNER et de référence micro TRI-GLOSS. On obtient une valeur de la brillance moyenne comprise entre 0 et 100.

Les valeurs mesurées sont indiquées ci-après pour chacune des compositions testées.

| | Vernis transparent préparé avec la bentone B27 V commercialisée par Elementis | Vernis transparent préparé avec le phyllosilicate Nanomer I.34TCN commercialisé par Nanocor |
|---|---|---|
| Brillance 20°/60° | 54/79 | 71/87 |

## Revendications

1. Composition cosmétique de soin et/ou de maquillage de la peau, des lèvres et/ou des phanères comprenant, dans un milieu physiologiquement acceptable contenant au moins une phase grasse, au moins une quantité efficace de phyllosilicates exfoliés dérivant d'au moins un phyllosilicate intercalé par une ou plusieurs molécules d'un agent intercalant non polyphénolique.

2. Composition selon la revendication 1, **caractérisée en ce que** les phyllosilicates exfoliés possèdent un facteur de forme variant de 50 à 2000, notamment de 75 à 1500, et en particulier de 200 à 1000.

3. Composition selon la revendication 1 ou 2, **caractérisée en ce qu'**au moins 50 %, notamment 70 %, en particulier 90 %, voire 95 % de phyllosilicates exfoliés sont formés de moins de dix feuillets, notamment moins de 5 feuillets, en particulier moins de 3 feuillets, voire d'un seul feuillet de phyllosilicate.

4. Composition selon l'une quelconque des revendications précédentes, **caractérisée en ce qu'**elle possède une teneur en phyllosilicates exfoliés variant de 0,05 à 20 % en poids, notamment de 0,1 à 15 % en poids, et en particulier de 0,5 à 10 % en poids par rapport au poids total de la composition.

5. Composition selon l'une quelconque des revendications précédentes, **caractérisée en ce que** les phyllosilicates exfoliés dérivent de l'intercalation d'argiles minérales de type smectite.

6. Composition selon l'une quelconque des revendications précédentes, **caractérisée en ce que** les smectites sont choisis parmi les montmorillonites, nontronites, beidellites, volkonskoïtes, hectorites, saponites, sauconites, sobockites, stevensites, svinfordites, vermiculites et leurs mélanges.

7. Composition selon l'une quelconque des revendications précédentes, **caractérisée en ce que** les phyllosilicates exfoliés possèdent au moins 15 % en poids, notamment au moins 20 % en poids et plus particulièrement au moins 30 % en poids d'agent intercalant par rapport au poids de phyllosilicate sec.

8. Composition selon l'une quelconque des revendications précédentes, **caractérisée en ce que** l'agent intercalant est choisi parmi :
a) les oniums possédant au moins une chaîne hydrocarbonée en C₁ à C₅₀,
b) les composés organiques, de nature polymérique ou non, possédant au moins un cycle aromatique ou au moins un groupe polaire choisi parmi les groupes carboxyle, hydroxyle, polyol, acide carboxylique, aldéhyde, cétone, amine linéaire ou cyclique, amide linéaire ou cyclique, ester, lactone et éther.

9. Composition selon la revendication 8, **caractérisée en ce que** le cation onium est choisi parmi les ammoniums primaires, secondaires, tertiaires ou quaternaires possédant au moins une chaîne alkyle en C₄-C₅₀.

10. Composition selon la revendication 9, **caractérisée en ce que** le composé organique est un composé non polymérique possédant un groupe polaire et au moins une chaîne hydrophobe, notamment une chaîne alkyle en C₄ à C₅₀, alkylène en C₄ à C₅₀ et/ou alkylaryle en C₄ à C₅₀.

11. Composition selon la revendication 10, **caractérisée en ce que** le composé organique non polymérique est choisi parmi les alkylpyrrolidones avec une chaîne alkyle en C₄ à C₅₀, en particulier en C₈ à C₃₀.

12. Composition selon la revendication 8, **caractérisée en ce que** le composé organique est un oligomère ou polymère synthétique possédant au moins un noyau aromatique ou un groupe polaire et notamment choisi parmi les dérivés polyvinylpyrrolidones (PVP), les dérivés polyvinylalcools (PVA), les dérivés de polyalkylènes oxydes, les dérivés polyacryliques sous leur forme polymérique et copolymérique, les dérivés acides polyméthacryliques (PMAA), les dérivés polyvinyloxazolidones (PVO), les dérivés polyvinylméthyloxazolidones (PVMO) et les dérivés polyvinyloxazolines.

13. Composition selon l'une quelconque des revendications précédentes, **caractérisée en ce que** ledit phyllosilicate exfolié dérive de montmorillonite-PVP, montmorillonite-PVA, et montmorillonite-alkylpyrrolidone et leurs mélanges.

14. Composition selon l'une quelconque des revendications précédentes, **caractérisée en ce qu'**elle est sous forme d'une émulsion.

15. Composition cosmétique de soin et/ou de maquillage de la peau, des lèvres et/ou des phanères de type émulsion, ladite composition comprenant, dans un milieu physiologiquement acceptable contenant au moins une phase grasse, au moins une quantité efficace de phyllosilicates exfoliés dérivant d'au moins un phyllosilicate intercalé par une ou plusieurs molécules d'un agent intercalant et étant dépourvue d'agent émulsionnant et d'agent co-émulsionnant.

16. Composition selon la revendication 15, **caractérisée en ce que** les phyllosilicates sont tels que définis en revendications 2 à 13.

17. Composition selon l'une quelconque des revendications 14 à 16, **caractérisée en ce que** l'émulsion est de type directe ou inverse.

18. Composition selon l'une quelconque des revendications 14 à 17, **caractérisée en ce que** l'émulsion est de type eau-dans-huile ou huile-dans-eau ou multiple.

19. Composition selon l'une quelconque des revendications 14 et 17 à 18, **caractérisée en ce qu'**elle comprend un agent émulsionnant, et éventuellement un agent co-émulsionnant, en proportion inférieure à 30 % en poids, et en particulier inférieure à 20 % en poids, et plus particulièrement inférieure à 15 %, et en particulier en proportion inférieure à 0,5 % en poids par rapport au poids total de la composition.

20. Composition selon l'une quelconque des revendications 14 et 17 ou 18, **caractérisée en ce qu'**elle est dépourvue d'agent émulsionnant et d'agent co-émulsionnant:

21. Composition selon l'une quelconque des revendications précédentes, **caractérisée en ce qu'**elle comprend une phase grasse continue.

22. Composition selon l'une quelconque des revendications précédentes, **caractérisée en ce qu'**elle est anhydre.

23. Composition selon l'une quelconque des revendications précédentes, **caractérisée en ce que** ladite phase grasse contient au moins un corps gras liquide à température ambiante et à pression atmosphérique et/ou au moins un corps gras solide à température ambiante et à pression atmosphérique.

24. Composition selon la revendication 23, **caractérisée en ce que** ledit corps gras liquide à température ambiante et à pression atmosphérique comprend au moins une huile volatile ou non volatile ou un de leurs mélanges.

25. Composition selon la revendication 24, **caractérisée en ce que** l'huile non volatile est choisie parmi les huiles hydrocarbonées d'origine animale ; les huiles hydrocarbonées végétales ; les hydrocarbures linéaires ou ramifiés d'origine minérale ou synthétique ; les éthers de synthèse ayant de 10 à 40 atomes de carbone; les esters de synthèse, les esters de polyol ; les alcools gras liquides à température ambiante ayant de 12 à 26 atomes de carbone ; les acides gras supérieurs ; les huiles siliconées de type polyméthylsiloxanes (PDMS) et leurs mélanges.

26. Composition selon l'une quelconque des revendications 23 à 25, **caractérisée en ce que** le corps gras liquide comprend au moins une huile volatile choisie parmi les huiles hydrocarbonées ayant de 8 à 16 atomes de carbone et les huiles de silicones volatiles.

27. Composition selon l'une quelconque des revendications 23 à 26, **caractérisée en ce que** ledit corps gras liquide à température ambiante et à pression atmosphérique représente de 0,01 à 90 %, notamment de 0,1 à 85 %, en poids par rapport au poids total de la phase grasse.

28. Composition selon l'une quelconque des revendications 23 à 27, **caractérisée en ce que** ledit corps gras solide à température ambiante et à pression atmosphérique est choisi parmi les cires, les corps gras pâteux, les gommes et leurs mélanges.

29. Composition selon l'une quelconque des revendications 23 à 28, **caractérisée en ce que** ledit corps gras solide représente de 0,01 à 50 %, notamment de 0,1 à 40 %, et en particulier de 0,2 à 30 %, en poids par rapport au poids total de la composition.

30. Composition selon l'une quelconque des revendications précédentes, **caractérisée en ce que** ladite composition comprend en outre une phase particulaire à raison de 0,01 à 40 %, notamment de 0,01 à 30 %, et en particulier de 0,05 à 20 %, en poids par rapport au poids total de ladite composition.

31. Composition selon la revendication 30, **caractérisée en ce que** ladite phase particulaire comprend au moins un pigment et/ou au moins une nacre et/ou au moins une charge complémentaire.

32. Composition selon l'une quelconque des revendications précédentes, **caractérisée en ce que** ladite composition comprend en outre un polymère filmogène.

33. Composition selon la revendication 32, **caractérisée en ce que** le polymère filmogène est choisi parmi les polymères filmogènes liposolubles, les polymères filmogènes lipodispersibles et leurs mélanges.

34. Composition selon l'une quelconque des revendications précédentes, **caractérisée en ce qu'**elle se présente sous la forme de produits coulés en stick ou en coupelle.

35. Composition selon l'une quelconque des revendications précédentes, **caractérisée en ce qu'**elle se présente sous la forme de rouges à lèvres, baumes à lèvres, fonds de teint coulés, produits anti-cernes, produits « correcteurs » ou « embellisseurs » de teint, et/ou fards à paupières ou à joues.

36. Composition selon l'une quelconque des revendications 1 à 33, **caractérisée en ce qu'**elle se présente sous la forme d'une composition de soin et/ou de maquillage des ongles naturels ou synthétiques.

37. Utilisation de phyllosilicates exfoliés selon l'une quelconque des revendications 1 à 13 à titre d'agent pour texturer la phase grasse d'une composition cosmétique de soin et/ou de maquillage de la peau, des lèvres et/ou des phanères.

38. Utilisation selon la revendication 37, **caractérisée en ce que** la composition cosmétique est une émulsion.

39. Utilisation de phyllosilicates exfoliés selon l'une quelconque des revendications 1 à 13 à titre d'agent stabilisant d'une émulsion cosmétique.

40. Utilisation de phyllosilicates exfoliés tels que définis selon l'une quelconque des revendications 1 à 13 à titre d'agent pour ajuster les propriétés de tenue, la non migration et/ou le non transfert d'une composition cosmétique de maquillage et/ou de soin de la peau, des lèvres et/ou des phanères.

41. Utilisation dans une composition de soin et/ou de maquillage de la peau, de phyllosilicates exfoliés selon l'une quelconque des revendications 1 à 13, à titre d'agent pour matifier, lisser et/ou uniformiser le teint et/ou à estomper les défauts du relief de la peau.

42. Procédé de maquillage de la peau, des lèvres et/ou des phanères comprenant l'application sur la peau, les lèvres et/ou les phanères d'au moins une couche d'une composition telle que définie selon l'une quelconque des revendications 1 à 36.

43. Support synthétique maquillé sur lequel est présent en tout ou partie de sa surface une composition telle que définie selon l'une quelconque des revendications 1 à 36.
